(19) Europäisches Patentamt
European Patent Office
Office européen des brevets

(11) EP 2 396 320 B1

(12) FASCICULE DE BREVET EUROPEEN

(45) Date de publication et mention de la délivrance du brevet:
**15.05.2013 Bulletin 2013/20**

(21) Numéro de dépôt: **10708307.3**

(22) Date de dépôt: **09.02.2010**

(51) Int Cl.:
***C07D 407/14*** *(2006.01)* ***A61K 31/496*** *(2006.01)*
***A61P 3/00*** *(2006.01)*

(86) Numéro de dépôt international:
**PCT/FR2010/050207**

(87) Numéro de publication internationale:
**WO 2010/092289 (19.08.2010 Gazette 2010/33)**

(54) **DERIVES DE 3-BENZOFURANYL-INDOL-2-ONE SUBSTITUES EN POSITION 3, LEUR PREPARATION ET LEUR APPLICATION EN THERAPEUTIQUE**

IN DER 3-STELLUNG SUBSTITUIERTE 3-BENZOFURANYLINDOL-2-ONDERIVATE, DEREN HERSTELLUNG UND DEREN THERAPEUTISCHE VERWENDUNG

3-BENZOFURANYL-INDOL-2-ONE DERIVATIVES SUBSTITUTED AT THE 3 POSITION, PREPARATION THEREOF, AND THERAPEUTIC USE THEREOF

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**
Etats d'extension désignés:
**AL BA RS**

(30) Priorité: **12.02.2009 FR 0900622**

(43) Date de publication de la demande:
**21.12.2011 Bulletin 2011/51**

(73) Titulaire: **SANOFI**
**75008 Paris (FR)**

(72) Inventeurs:
- **BARONI, Marco**
**F-75013 Paris (FR)**
- **PULEO, Letizia**
**F-75013 Paris (FR)**

(74) Mandataire: **Veinante, Aude**
**Sanofi**
**Département Brevets**
**54, rue La Boétie**
**75008 Paris (FR)**

(56) Documents cités:
**WO-A-2005/035498 WO-A-2009/056707**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 396 320 B1

## Description

La présente invention a pour objet des dérivés de 3-benzofuranyl- indol-2-one substitués en 3, leur préparation et leur application en thérapeutique.

La ghréline est une hormone peptidique de 28 acides aminés produite de façon principale dans l'estomac par un processus post-traductionnel après clivage de la pré-pro-ghréline (Kojima M, et al., Nature 1999; 402:656-60). La ghréline est un ligand endogène du récepteur hypophysaire des sécrétagogues de l'hormone de croissance (GHSR1a).

GHS-R est codé par deux exons : l'exon 1 code pour les domaines transmembranaires (TMs) 1-5 et l'exon 2 code pour TM6 et 7 du récepteur couplé à la protéine G (GPCR).

Les deux transcripts ont été identifiés dans la glande pituitaire et le cerveau : l'un codant pour le GPCR de longueur totale (GHS-R1a) et l'autre codant pour un récepteur tronqué (GHS-R1b) auquel manquent TM6 et 7. Seul le sous-type GHS-R1a est activé par la ghréline et les mimétiques de la ghréline. GHS-R1 b est présent dans le foie et d'autres tissus périphériques mais sa fonction est inconnue (Smith R.G. et al, Trends in Endocrinology and Metabolism, 2005, 16 No. 9).

C'est un récepteur de type rhodopsine, à sept domaines transmembranaires de la famille A couplé à Gq/phospholipase C. Le récepteur de la ghréline peut aussi être couplé aux voies Gs/protéine kinase A dans certains tissus (Ueno, N. et al. Endocrinology, 2004, 145, 4176-4184 ; Kim, M.S. et al., Int. J. Obes. Relat. Metab. Disord., 2004, 28: 1264-1271). De façon intéressante, le récepteur de la ghréline présente la caractéristique peu commune de présenter une activité constitutive indépendante du ligand significative (Barazzoni, R. et al., Am. J. Physiol. Endocrinol. Metab., 2004, 288: E228-E235).

Des niveaux faibles de l'expression de la ghréline ont été documentés dans des tissus variés, tels que les intestins, le pancréas, les reins, le système immunitaire, le placenta, les testicules, les tissus pituitaires, l'hypothalamus (Horm. Res. 2003; 59 (3):109-17).

Il a été démontré que la ghréline est impliquée dans la faim au moment des repas, et dans l'initiation des repas. Les niveaux circulants diminuent avec la prise d'alimentation et augmentent avant les repas, atteignant des concentrations suffisantes pour stimuler la faim et la prise de nourriture. Les ingestions de ghréline stimulent la prise de nourriture rapidement et de façon transitoire, principalement en augmentant les comportements d'alimentation appétante et le nombre de repas. La ghréline stimule la prise de nourriture à court terme de façon plus efficace que n'importe quelle autre molécule, à l'exception du neuropeptide Y, avec lequel elle est approximativement équipotente (Wren A.M., et al., J. Clin. Endocrinol. Metab., 2001;86:5992-5.). Cependant, la ghréline est unique dans sa capacité à exercer cet effet, qu'elle soit injectée de façon périphérique ou centrale.

C'est également la seule substance de mammifère qui a démontré sa capacité à augmenter l'appétit et la prise d'alimentation lorsqu'elle est administrée aux humains (Druce M.R., et al., Int. J. Obes., 2005; 29:1130-6; Wynne K., et al., J. Am. Soc. Nephrol., 2005; 16: 2111-8.).

Au-delà de son rôle dans l'initiation des repas, la ghréline remplit également les critères établis d'une hormone liée à l'adiposité impliquée dans la régulation de la masse corporelle à long terme. Les taux de ghréline circulent en fonction des stocks énergétiques et manifestent des changements compensatoires en réponse aux modifications de la masse corporelle.

La ghréline traverse la barrière hémato-encéphalique et stimule la prise de nourriture en agissant sur certains centres régulateurs classiques de la masse corporelle, tels que l'hypothalamus, le cerveau postérieur et le système compensatoire mésolimbique.

L'administration chronique de ghréline augmente la masse corporelle par diverses actions concertées sur la prise de nourriture, la dépense énergétique et l'utilisation des ressources. L'ablation congénitale de la ghréline ou du gène du récepteur de la ghréline provoque une résistance à l'obésité induite par l'alimentation, et le blocage pharmacologique de la ghréline réduit la prise de nourriture et la masse corporelle.

Les preuves existantes semblent favoriser le rôle de la ghréline à la fois dans l'initiation des repas à court terme et l'homéostasie énergétique à long terme, en faisant ainsi une cible attractive comme médicament pour traiter l'obésité et/ou les désordres de l'amaigrissement.

La ghréline exerce également des actions à la fois physiologiques et pharmacologiques sur le pancréas endocrine. La ghréline bioactive acylée est produite dans la cellule-$\varepsilon$, récemment décrite dans les îlots pancréatiques (Prado, C.L., et al., 2004, Proc. Natl. Acad. Sci. USA, 101:2924-2929), fournissant potentiellement une source locale de ghréline qui agit sur les cellules $\beta$ des îlots. Le blocage de cette fonction de la ghréline endogène par le biais d'un antagoniste pour ses récepteurs a diminué les concentrations de glucose à jeûn de façon prononcée, a atténué le mouvement glycémique et augmenté les réponses à l'insuline lors des tests à la tolérance au glucose, suggérant un rôle inhibiteur pour la ghréline dans le contrôle de la sécrétion de l'insuline (Dezaki, K., et al. 2004, Diabètes, 53: 3142-3151).

L'ablation de la ghréline chez les souris (souris ghréline -/-) augmente la sécrétion d'insuline dépendante du glucose par la cellule $\beta$ du pancréas par réduction de l'expression Ucp2 et améliore la sensibilité à l'insuline périphérique

(Sun Y. et al., 2006, Cell Metabolism, 3:379-386).

Des antagonistes du récepteur de la ghréline pourraient ainsi réguler la faim, la prise de repas et leur fréquence, ainsi que, à long terme, le poids, notamment la prise de poids faisant suite aux régimes diététiques ou thérapeutiques. De plus, dans le cadre d'un traitement antidiabétique, les antagonistes de la ghréline pourraient être utiles pour maintenir l'équilibre entre insuline et glucose et pour contrôler l'hyperphagie diabétique. Les antagonistes de la ghréline pourraient ainsi être utilisés comme agents anorexiques et/ou anti-obésité, ou encore dans le traitement du diabète et de ses effets.

La présente invention a pour objet les composés répondant à la formule (I) :

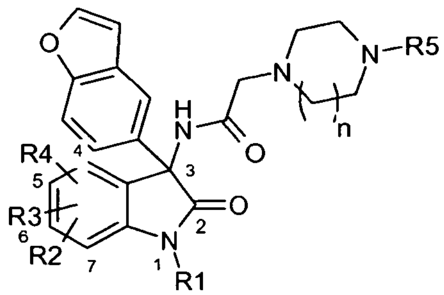

(I)

dans laquelle :

R1 représente un atome d'hydrogène ou un groupe (C1-6) alkyle, -C(=O)(C1-6) alkyle, -C(=O)aryle;

R2, R3, R4, identiques ou différents, situés sur l'une quelconque des positions disponibles du noyau phényle, représentent indépendamment un atome d'hydrogène, un atome d'halogène, CN , OH, un groupe (C1-6) alkyle éventuellement substitué par un atome d'halogène ou un OH ; perhalogéno(C1-3) alkyle, (C1-6) alcoxy, perhalogéno (C1-3) alcoxy , aminocarbonyle, (C1-6) alkylaminocarbonyle, di(C1-6) alkylaminocarbonyle, aryle, aryloxy ; hétéroaryle ; le groupe aryle, aryloxy ou hétéroaryle pouvant éventuellement être substitué par un atome d'halogène, CN, OH ou un groupe (C1-6) alkyle , perhalogéno(C1-3) alkyle ou (C1-6) alcoxy ; étant entendu que au moins un des R2, R3, R4 est différent de H et que le groupe aryle, aryloxy ou hétéroaryle peut éventuellement être substitué par un atome d'halogène, CN, OH ou un groupe (C1-6) alkyle , perhalogéno(C1-3) alkyle, (C1-6) alcoxy;

R5 représente un groupe (C1-6) alkyle ou un (C2-6) alcényle;et

n représente 1 ou 2.

Les composés de formule (I) comportent un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Dans le cadre de la présente invention, on entend par :

- un atome d'halogène : un fluor, un chlore, un brome ou un iode ;
- un groupe alkyle : un groupe aliphatique saturé linéaire ou ramifié. A titre d'exemples, on peut citer un groupe (C1-6) alkyle comprenant de 1 à 6 atomes de carbone, plus particulièrement (C1-4) alkyle qui peut représenter un méthyle, éthyle, propyle, isopropyle, butyle, isobutyle, tertbutyle ;
    - un groupe alcényle : un groupe aliphatique mono- ou poly-insaturé, linéaire ou ramifié, comprenant par exemple une ou deux insaturations et comprenant de 2 à 6 atomes de carbone ;
    - un groupe halogénoalkyle un groupe alkyle dont un ou plusieurs atomes d'hydrogène ont été substitués par un atome d'halogène ; par exemple un fluoroalkyle : un groupe alkyle dont un ou plusieurs atomes d'hydrogène ont été substitués par un atome de fluor ;
- un groupe perhalogénoalklyle : un groupe alkyle dont tous les atomes d'hydrogène ont été substitués par un atome d'halogène ; par exemple un perfluoroalkyle : un groupe alkyle dont tous les atomes d'hydrogène ont été substitués

par un atome de fluor ;

- un groupe alcoxy : un radical -O-alkyle où le groupe alkyle est tel que précédemment défini;
- un groupe perhalogénoalcoxy : un radical -O-perhalogénoalkyle où le groupe perhalogénoalkyle est tel que précédemment défini, à titre d'exemple on peut citer le trifluorométhoxy;
- un groupe aryle : un groupe aromatique cyclique comprenant entre 6 et 10 atomes de carbone. A titre d'exemples de groupes aryles, on peut citer un phényle ou naphthyle;
- un groupe hétéroaryle : un groupe aromatique cyclique comprenant entre 2 et 10 atomes de carbone et comprenant entre 1 et 3 hétéroatomes, tels que l'azote, l'oxygène ou le soufre. A titre d'exemples de groupes hétéroaryles, on peut citer les groupes furanyle, pyrrolyle, imidazolyle, pyrazolyle, thiényle, oxadiazolyle, oxazolyle, isoxazolyle, furazanyle, thiadiazolyle, thiazolyle, isothiazolyle, pyridyle, pyrazinyle, pyrimidinyle, pyridazinyle ainsi que les groupes correspondants résultants de la fusion avec un groupe phényle tel que par exemple benzothiophène, benzofurane; benzothiazole, ...

Parmi les composés de formule (I) objets de l'invention, un groupe de composés est constitué par les composés pour lesquels :

R1 représente un atome d'hydrogène ou un groupe (C1-6) alkyle, -C(=O)(C1-6) alkyle, -C(=O)aryle;
R2, R3, R4, identiques ou différents, situés sur l'une quelconque des positions disponibles du noyau phényle, représentent indépendamment un atome d'hydrogène, un atome d'halogène, CN , OH, un groupe (C1-6) alkyle, perhalogéno(C1-3) alkyle, (C1-6) alcoxy, perhalogéno(C1-3) alcoxy , aminocarbonyle, (C1-6) alkylaminocarbonyle, di(C1-6) alkylaminocarbonyle, aryle, aryloxy , hétéroaryle, étant entendu que au moins un des R2, R3, R4 est différent de H ;
R5 représente un groupe (C1-6) alkyle ;
n représente 1 ou 2 ;

sous forme de base ou de sel d'addition à un acide.

Parmi les composés de formule (I) objets de l'invention, un groupe de composés est constitué par les composés pour lesquels :

R1 représente un atome d'hydrogène ou un groupe -C(=O)(C1-6) alkyle, -C(=O)aryle, (C1-6) alkyle ; et/ou
R2, R3, R4, identiques ou différents, situés sur l'une quelconque des positions disponibles du noyau phényle, représentent indépendamment un atome d'hydrogène, un atome d'halogène, plus particulièrement le chlore ou le brome, ou un groupe (C1-6) alkyle ou trifluorométhyle, étant entendu que au moins un des R2, R3, R4 est différent de H ; et/ou
R5 représente un groupe (C1-6) alkyle ; et/ou
n représente 1 ou 2 ;

sous forme de base ou de sel d'addition à un acide.

Parmi les composés de formule (I) objets de l'invention, un autre groupe de composés est constitué par les composés pour lesquels :

R1 représente un atome d'hydrogène ou un groupe -C(=O)méthyle, -C(=O)phényle, méthyle ; et/ou
R2, R3, R4, identiques ou différents, situés sur l'une quelconque des positions disponibles du noyau phényle, représentent indépendamment un atome d'hydrogène, un atome d'halogène, plus particulièrement le chlore ou le brome, ou un groupe méthyle ou trifluorométhyle, étant entendu que au moins un des R2, R3, R4 est différent de H ; et/ou
R5 représente un groupe méthyle, éthyle ou 2-propyle; et/ou
n représente 1 ou 2 ;

sous forme de base ou de sel d'addition à un acide.

Parmi les composés de formule (I) objets de l'invention, on peut notamment citer le composé suivant :

Composé n°1 : (+) N-[4,6-Dichloro-3-(benzofuran-5-yl)-2-oxo-2,3-dihydro-1H-indol-3-yl]-2-(4-éthyl-pipérazin-1-yl)-acétamide ;

sous forme de base ou de sel d'addition à un acide.

Dans ce qui suit, on entend par groupe protecteur Pg un groupe qui permet, d'une part, de protéger une fonction réactive telle qu'un hydroxy ou une amine pendant une synthèse et, d'autre part, de régénérer la fonction réactive intacte

en fin de synthèse. Des exemples de groupes protecteurs ainsi que des méthodes de protection et de déprotection sont données dans Protective Groups in Organic Synthesis, Green et al., 2nd Edition (John Wiley & Sons, Inc., New York).

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans Advances in Organic Chemistry, J. March, 3rd Edition, Wiley Interscience, p. 310-316.

Conformément à l'invention, on peut préparer les composés de formule générale (I) selon le procédé qui suit :

Schéma 1 :

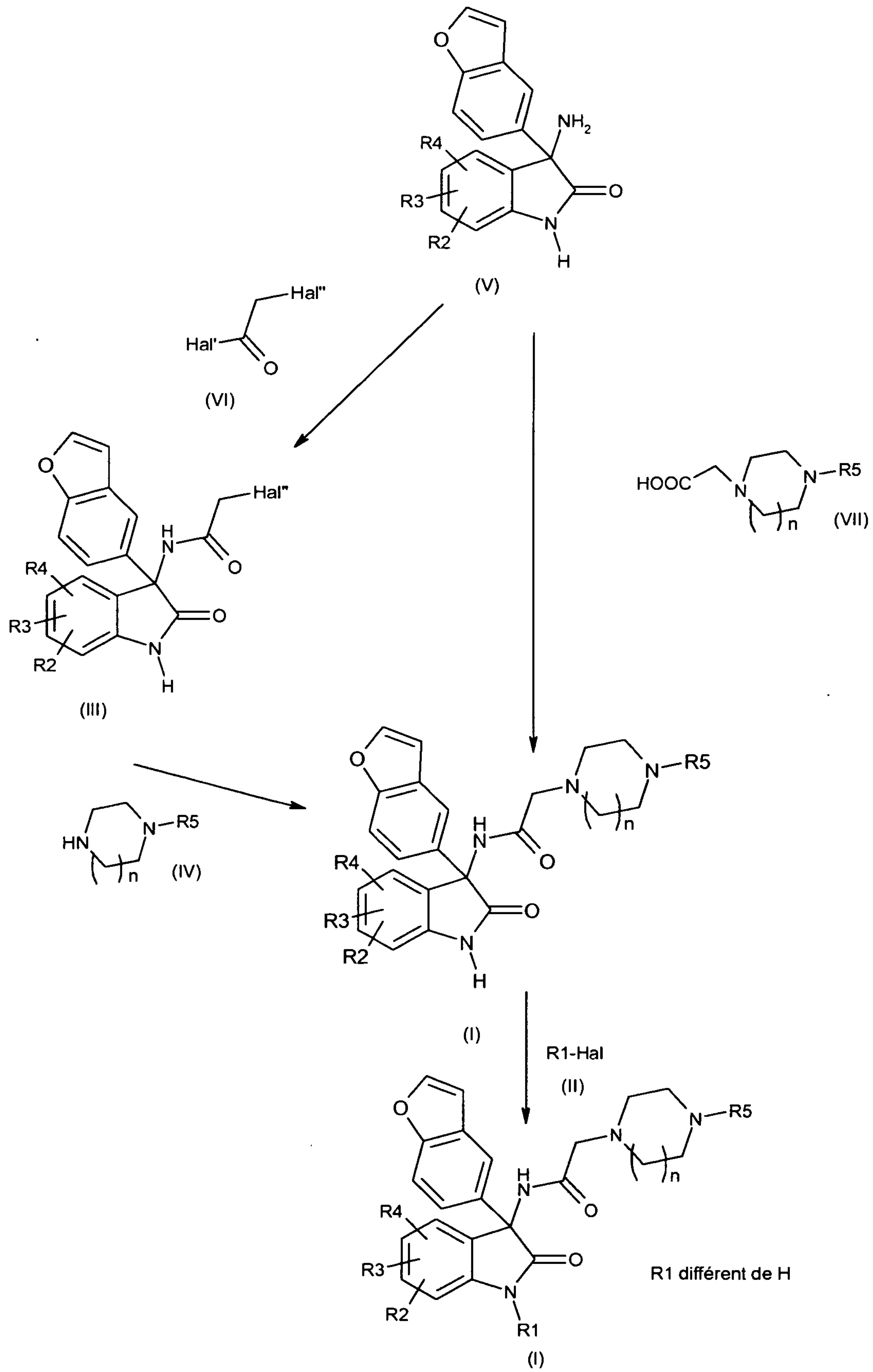

Le composé de formule (I), dans laquelle R1 est différent de H, R2, R3, R4, R5, et n sont tels que définis en formule générale (1) peut être préparé par réaction d'un composé de formule (I) dans laquelle R1=H avec un composé de formule (II) :

R1-Hal (II)

dans laquelle R1, différent de H est défini comme en formule générale ()I et Hal représente un atome d'halogène, par exemple le chlore selon des méthodes connues de l'homme du métier par exemple en présence d'une base, telle que $K_2CO_3$, NaH, t-$BuO^-K^+$, dans un solvant tel que le diméthylformamide (DMF), tétrahydrofurane (THF), diméthoxyéthane, le diméthylsulfoxide (DMSO).

Le composé de formule générale (I) dans laquelle R1=H peut être préparé selon l'une ou l'autre des variantes suivantes :

Par réaction d'un composé de formule générale (III) :

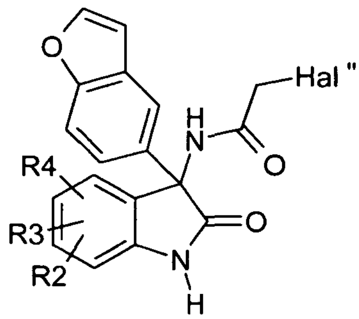

(III)

avec un composé de formule générale (IV) :

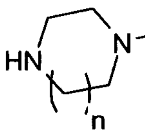

(IV)

dans lesquelles, R2, R3, R4, R5 et n sont tels que définis en formule générale (I) et Hal" représente un atome d'halogène, de préférence le chlore. Cette réaction est généralement effectuée au moyen d'une base, organique ou minérale, telle que $K_2CO_3$, $Na_2CO_3$, la pyridine ou 4-diméthylaminopyridine, en présence de NaI ou KI, dans un solvant inerte tel que le DMF, le dichlorométhane, le THF, le diméthoxyéthane ou le toluène.

Le composé de formule générale (III) peut être préparé à partir d'un composé de formule générale (V) :

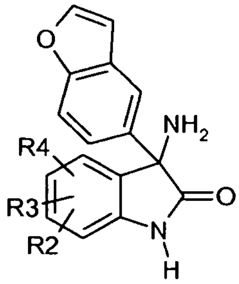

(V)

et d'un composé de formule générale (VI) :

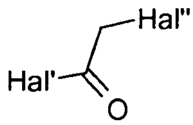

(VI)

dans lesquelles R2, R3 et R4, sont tels que définis en formule générale (I) et Hal’ et Hal", identiques ou différents représentent indépendamment un atome d’halogène, de préférence le chlore.

Cette réaction est généralement effectuée au moyen de pyridine ou 4-diméthylaminopyridine dans un solvant tel que le toluène, benzène ou dichlorométhane, préférentiellement à température comprise entre la température ambiante et la température de reflux du solvant.

Par température ambiante on entend une température comprise entre 5 et 25°C.

Le composé de formule générale (I) dans laquelle R1=H peut également être préparé à partir d’un composé de formule générale (V) :

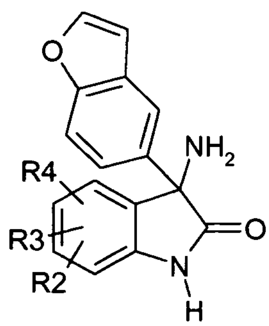

(V)

et d’un composé de formule générale (VII) :

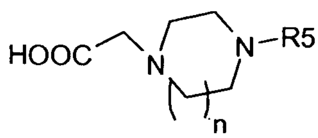

(VII)

dans lesquelles, R2, R3, R4, R5 et n sont tels que définis en formule générale (I). Cette réaction est généralement effectuée au moyen d’un agent d’halogénation, tel qu’un agent de chloration, par exemple les chlorures de phosphore, notamment $PCl_5$, ou encore $PCl_3$ ou $POCl_3$. La réaction est généralement réalisée en présence de pyridine ou 4-diméthylaminopyridine, dans un solvant tel que le dichlorométhane ou le DMF.

Les intermédiaires de formule générale (V) sont connus et peuvent être préparés selon les procédés illustrés par le schéma qui suit :

Schéma 2 :

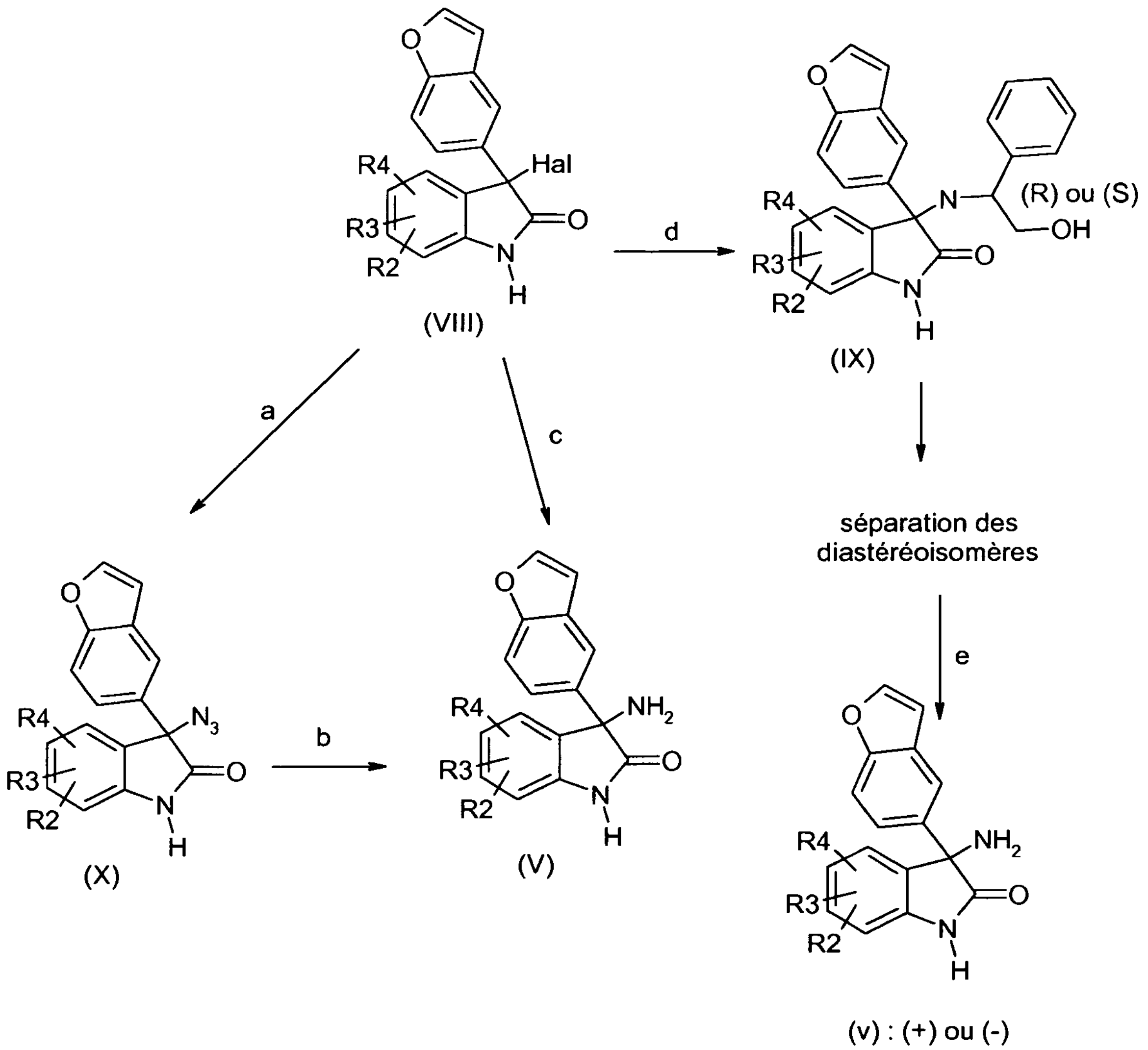


(VIII) (IX) (X) (V) (v) : (+) ou (-)

dans lequel R2, R3 et R4 sont tels que définis en formule générale (I) et Hal représente un atome d'halogène, par exemple le chlore.

A l'étape c du Schéma 2, on prépare un composé de formule (V) à partir d'un composé de formule (VIII) par barbotage d'ammoniac gazeux selon la méthode décrite dans la demande FR 2 714 378.

On peut également préparer le même composé par réduction d'un composé de formule (X) selon des méthodes connues de l'homme du métier, par exemple au moyen du zinc dans un solvant tel que le méthanol. La préparation d'un composé de formule (X) de l'étape est décrite dans la demande FR 2 714 378.

Un composé de formule (V) optiquement pur peut être synthétisé selon les étapes d et e du Schéma 2, telles que décrites dans la demande WO 03/008407.

Les intermédiaires de formule générale (VIII) peuvent être préparés selon le procédé décrit dans la demande WO 03/008407 et illustrés par le schéma 3 :

Schéma 3 :

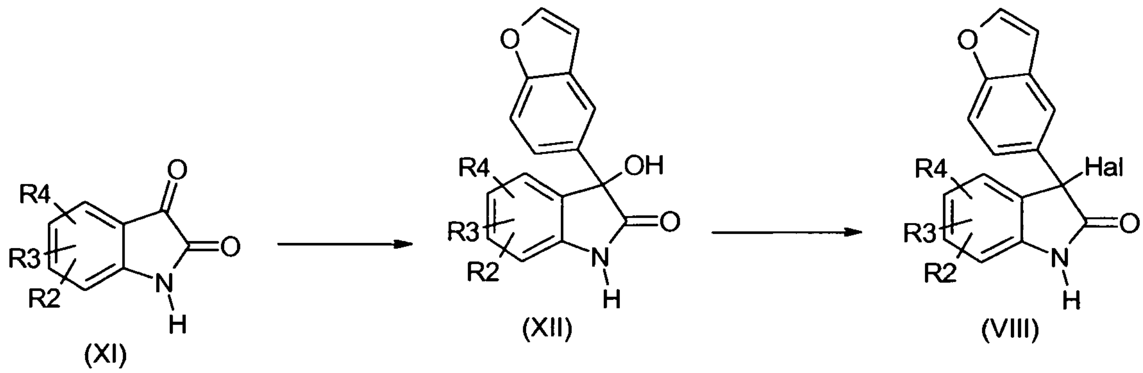


dans lequel R2, R3 et R4 sont tels que définis en formule générale (I) et Hal représente un atome d'halogène, par exemple le chlore.

Le composé de formule générale (VII) peut être préparé selon la méthode suivante, illustrée par le schéma 4 :

Schéma 4 :

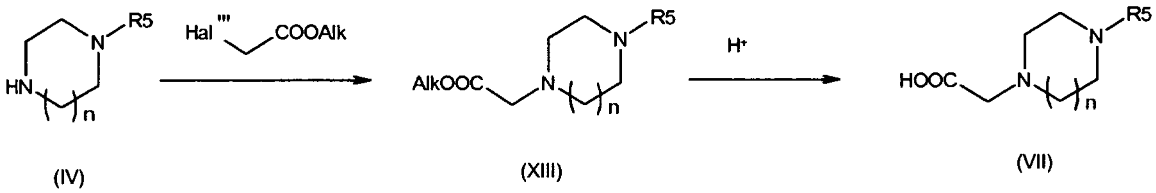


Le composé de formule générale (XIII) peut être préparé par condensation d'un composé de formule générale (IV) :

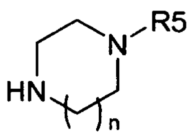

(IV)

dans laquelle R5 et n sont définis comme en formule générale (I), avec un composé halogéné correspondant, tel que Hal'''CH2COOAlk, où Hal''' représente un atome d'halogène, tel que le chlore et Alk représente un groupe alkyle, tel que éthyle.

Cette réaction est avantageusement effectuée dans un solvant tel que le toluène ou le benzène ou le dioxane.

Selon un autre mode de réalisation les composés de formule générale (I) dans laquelle R1 représente un groupe alkyle et R2, R3, R4, R5, et n sont tels que définis en formule générale (I) peuvent également être préparés selon le schéma 5 suivant :

Schéma 5 :

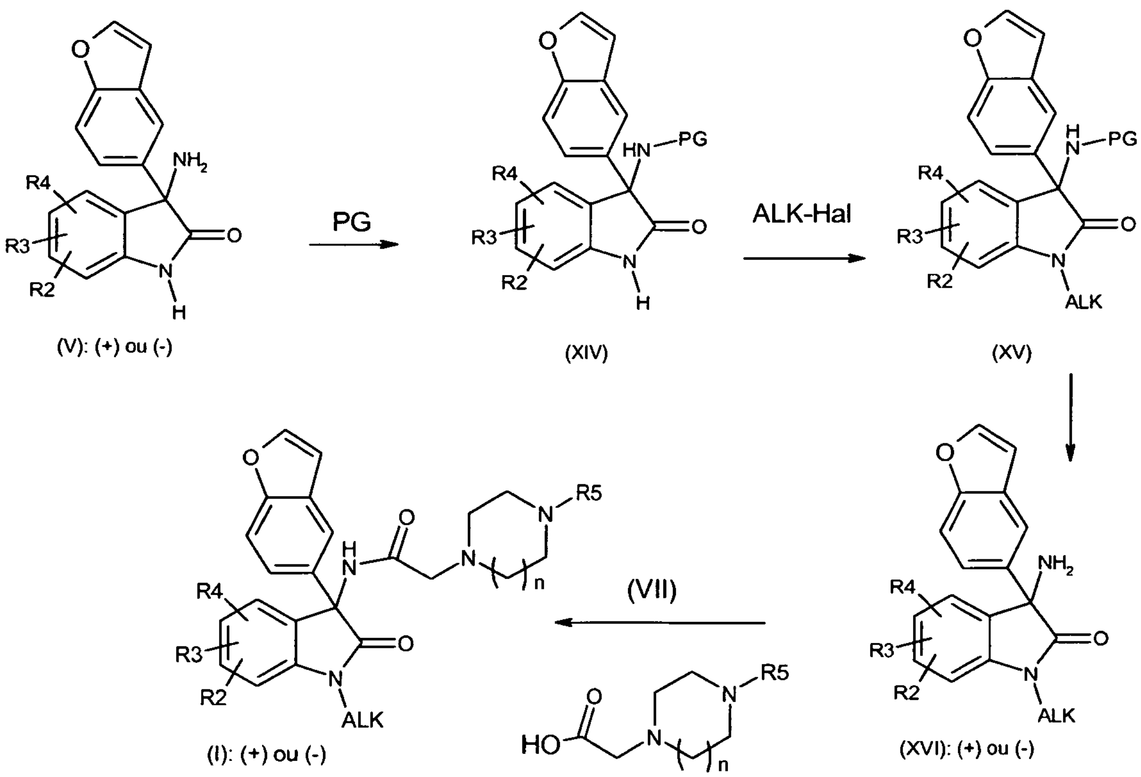

(V): (+) ou (-)
(XIV)
(XV)
(XVI): (+) ou (-)
(I): (+) ou (-)

Selon ce schéma, on fait réagir un composé de formule (V) avec un groupe protecteur PG pour donner le composé de formule (XIV). En tant que groupe protecteur PG de l'amine, on peut utiliser par exemple la benzimine ou le t-butyl carbamate. Ces derniers sont introduits selon des méthodes connues de l'homme du métier par exemple en présence d'une base, telle que $K_2CO_3$, NaOH, triéthylamine dans un solvant tel que le dioxane, THF ou le DMSO.

Le composé de formule générale (XV) peut être préparé par réaction d'un composé de formule (XIV) avec un composé ALK-Hal dans laquelle ALK représente un groupe aliphatique saturé linéaire ou ramifié comprenant de 1 à 6 atomes de carbone et Hal représente un atome d'halogène, par exemple le chlore.

On obtient le composé de formule générale (XVI) à partir d'un composé de formule (XV) par élimination du groupe protecteur selon des méthodes bien connues, par exemple en milieu acide par HCl ou acide trifluoroacétique.

On fait ensuite réagir avec un composé de formule générale (VII) :

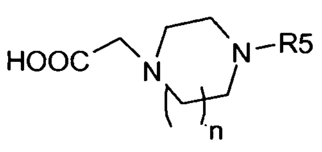

(VII)

dans laquelle R5 et n sont tels que définis en formule générale (I). Cette réaction est généralement effectuée au moyen d'un agent d'halogénation, tel qu'un agent de chloration, par exemple les chlorures de phosphore, notamment $PCl_5$, ou encore $PCl_3$ ou $POCl_3$. La réaction est généralement réalisée en présence de pyridine ou 4-diméthylaminopyridine, dans un solvant tel que le dichlorométhane ou le DMF.

Eventuellement, on transforme le composé de formule (I) en l'un de ses sels d'addition à un acide.

Le procédé selon l'invention peut éventuellement comprendre l'étape consistant à isoler le produit de formule générale (I) désiré.

Dans les schémas 1, 2, 3, 4 et 5 les composés de départ et les réactifs, quand leur mode de préparation n'est pas décrit, sont disponibles dans le commerce ou décrits dans la littérature, ou bien peuvent être préparés selon des

méthodes qui y sont décrites ou qui sont connues de l'homme du métier.

L'invention, selon un autre de ses aspects, a également pour objet les composés de formule (III). Ces composés sont utiles comme intermédiaires de synthèse des composés de formule (I).

L'invention, selon un autre de ses aspects, a également pour objet les composés de formule (XVI). Ces composés sont utiles comme intermédiaires de synthèse des composés de formule (I).

Les exemples suivants décrivent la préparation de certains composés conformes à l'invention. Ces exemples ne sont pas limitatifs et ne font qu'illustrer la présente invention.

Les mesures physico-chimiques ont été effectuées de la façon suivante : Les points de fusion ont été mesurés avec un appareil BUCHI B-540.

Les spectres de résonance magnétique nucléaire du proton (RMN 1 H) ont été enregistrés à 500 MHz sur un appareil Bruker équipé avec une console Avance III. Les déplacements chimiques sont rapportés en ppm par rapport à la fréquence du TMS.

Touts les spectres ont été enregistrés à la température de 40°C.

Les abréviations utilisées pour caractériser les signaux sont les suivantes: s = singulet, bs =singulet large, m = multiplet, bm= multiplet large, d = doublet, bd= doublet large, t = triplet, q = quadruplet.

* = non intégrable à cause de l'interférence avec un pic large du à l'eau.
** = non intégrable à cause de l'interférence avec un pic du au solvant RMN.
***= lu au premier ordre.
****= diastéréoisomère le plus abondant.
*****= diastéréoisomère le moins abondant.

Les conditions d'analyse par chromatographie liquide couplée à la spectrométrie de masse (LC/UV/MS) sont les suivantes:

Pour la partie chromatographie liquide.
Méthode A
Colonne Kromasil C18 3,5 μm

- Eluant A = $H_2O$ + 0,01 % TFA
- Eluant B = $CH_3CN$.
- Gradient de 98% de A à 95% de B en 10 minutes, puis élution par 95% de B pendant 5 minutes.
- Débit 0,3ml/minute
- Injection de 2μL de solution à 0,1mg/ml dans un mélange $CH_3CN$ : $H_2O$ = 9 :1

Méthode B
Colonne XTerra MS C18 x 50 3,5 μm

- Eluant A = $H_2O$ + 0,01% TFA
- Eluant B = $CH_3CN$.
- Gradient de 98% de A à 95% de B en 10 minutes, puis élution par 95% de B pendant 5 minutes.
- Débit 0,5 ml/minute
- Injection de 2μL de solution à 0,1mg/ml dans un mélange $CH_3CN$ : $H_2O$ = 9 :1

Les produits sont détectés en UV à 220 nm.

Pour la partie spectrométrie de masse :

- Mode d'ionisation: électrospray positif (API-ES polarité+)
- Balayage de 100 à 1200 uma

La chromatographie en couche mince a été effectuée sur des plaques CCM de gel de silice de Merck. Le gel de silice pour la chromatographie sur colonne flash est commercialisé par Biotage.

Tous les solvants utilisés sont de pureté "reagent grade" ou "HPLC grade".

Les mesures de Alpha D ont été effectuées sur un polarimétre Perkin Elmer modéle PE341 en utlilisant une cellule avec un chemin optique de 1 dm.

Dans les exemples et préparations :

AcOH et AcOEt représentent respectivement l'acide acétique et l'acétate d'éthyle.

MeOH, EtOH, tBuOH représentent respectivement méthanol, éthanol et ter-butanol.
THF représente le tétrahydrofurane
Pf représente le point de fusion.

Préparation 1 : **Acide (4-éthyl-pipérazin-1-yl) acétique**

(i) éthyle (4-éthyl)-pipérazin-1-yl)-acétate

Dans un ballon, on charge 8,9 ml d'éthylpipérazine dans 91,5 ml de toluène. On ajoute goutte à goutte une solution de 4,1 ml de bromoacétate d'éthyle dans 11,6 ml de toluène. On laisse réagir à reflux à 110°C pendant une heure, on concentre à petit volume et on laisse au frigo pendant 3 heures. Il se forme un précipité blanc qui est filtré et lavé avec du dichlorométhane. On évapore les eaux de filtration; on obtient 7 g de produit attendu.
CCM : AcOEt 1/ MeOH 1, Rf=0, 45

(ii) Acide (4-éthyl-pipérazin-1-yl) acétique

On ajoute 7 g du produit obtenu à l'étape précédente à 190 ml de HCl 6N et on laisse réagir 4 heures à reflux. On évapore à sec, on lave avec un mélange AcOEt 1 /EtOH 1 et on sèche le solide blanc obtenu. On obtient 7 g de produit attendu.
CCM: 100% MeOH, Rf=0, 2

Préparation 2:

**(+) 3-Amino-4,6-dichloro-1,3-dihydro-3-(benzofuran-5-yl)-indole-2-one**

(i) 3-Hydroxy-4,6-dichloro-1,3-dihydro-3-(benzofuran-5-yl)-indole-2-one

Dans un ballon muni d'un agitateur mécanique et sous flux d'azote, on charge 2, 25 g de magnésium pour Grignard dans 15 ml de THF anhydre. Puis on ajoute un mélange de 13, 6 g de 5-bromobenzofuranne dans 35 ml de THF anhydre. On laisse sous agitation pendant une heure, puis on ajoute une solution de 5 g de 4,6-dichloro-1H-indol-2,3-dione dans 50 ml de THF anhydre. On laisse agiter à température ambiante pendant 4 heures et demie. On ajoute de l'eau, et on extrait avec de l'acétate d'éthyle. On sépare la phase organique que l'on sèche sur $Na_2SO_4$, on filtre et on évapore sous vide. On reprend avec de l'acétate d'éthyle et on lave avec une solution de soude 1 N. La phase organique est séchée sur $Na_2SO_4$, filtrée et évaporée sous vide. Le solide est repris avec de l'éther éthylique et filtré. On obtient 4,2 g de produit attendu.
CCM: Hexane/EtOAc 6.4 , Rf=0, 35

(ii) 3,4,6-trichloro-1,3-dihydro-3-(benzofuran-5-yl)-indote-2-one

Dans un ballon muni d'un agitateur magnétique et sous flux d'azote, on charge 4,1 g du produit de l'étape précédente, dans 40 ml de dichlorométhane. A 0°C, on ajoute 1,7 ml de pyridine et un mélange de 1,4 ml de $SOCl_2$ dans 30 ml de dichlorométhane. On laisse réagir à température ambiante, puis on verse dans une solution aqueuse saturée de $NH_4Cl$. On sépare la phase organique qui est séchée sur $Na_2SO_4$, filtrée et évaporée sous vide.
CCM: Hex.7/ AcOEt 3, Rf=0, 65

(iii) 4,6-dichloro-[[(1S)-2-hydroxy-1-phényléthyl] amino] 1,3-dihydro -3-(benzofuran-5-yl)-indole-2-one isomère A et isomère B

Sous flux d'azote, on mélange 4,1 g du composé de l'étape précédente dans 50 ml de dichlorométhane et 3,1 g de S-phénylglycynol. On laisse réagir une nuit à température ambiante. On filtre le solide formé, les eaux de filtration sont évaporées à sec et purifiées sur colonne avec l'éluant Hex. /AcOEt 8:2.
On obtient 0, 64 g de produit moins polaire, isomère A (pf= 135°C) et 1,23 g de l'isomère plus polaire.

(iii) (+)-3-Amino-5,6-dichloro-1,3-dihydro-3-(4-chlorophényl)-indole-2-one

On met à réagir 1,21 g du produit obtenu dans l'étape précédente dans un mélange de 20 ml de dichlorométhane et 15 ml de méthanol. On ajoute 1,26 g de $Pb(OAc)_4$ et on laisse réagir à température ambiante pendant 1 heure. On évapore à sec et on reprend avec de l'acétate d'éthyle puis on lave avec une solution aqueuse saturée de $NaHCO_3$. La

phase organique est séchée, filtrée et concentrée. On reprend avec un mélange de 36 ml d'acide chlorhydrique 3N et 3,7 ml de méthanol et on laisse sous agitation pendant une nuit. On concentre et on dilue avec un mélange d'eau et de dichlorométhane. La phase organique est lavée avec une solution d'acide chlorhydrique 1 N. Les phases aqueuses sont réunies, portées à pH basique avec une solution de $NH_3$ aqueuse, et extraites avec du dichlorométhane. La phase organique est séchée, filtrée et concentrée pour obtenir 870 mg de produit solide blanc.
Pf=215-216°C
LC/MS : $(M+H)^+$ = m/z 333 uma; rt = 5,3 minutes

**Exemple 1**

**(+) N-[4,6-Dichloro-3-(benzofuran-5-yl)-2-oxo-2,3-dihydro-1H-indol-3-yl]-2-(4-éthyl-pipérazin-1-yl)-acétamide et son oxalate**

Méthode A:

(i) 2-Chloro-N-[4,6-dichloro-3-(benzofuran-5-yl)-2-oxo-2,3-dihydro-1H-indol-3-yl]-acétamide :
Dans un ballon muni d'un agitateur magnétique et sous flux d'azote, on charge 0,87 g du produit obtenu à la Préparation 2, 30 ml de toluène, 0,21 ml de pyridine et 0,21 ml de chlorure de chloroacétyle. On laisse réagir à 110°C pendant 4 heures, puis on verse le mélange réactionnel dans l'eau et on extrait à l'acétate d'éthyle. La phase organique est séchée sur $Na_2SO_4$, filtrée et évaporée sous vide. On obtient 900 mg de solide beige, purifié sur colonne par flash chromatographie au moyen du mélange cyclohexane 8/ acétate d'éthyle 2 pour obtenir 630 mg du produit attendu.

CCM: Hex. 1/ AcOEt 1. Rf=0. 5

(ii) (+) N-[4,6-Dichloro-3-(benzofuran-2-yl)-2-oxo-2,3-dihydro-1 H-indol-3-yl]-2-(4-éthyl-pipérazin-1-yl)-acétamide:
Dans un ballon muni d'un agitateur magnétique, on charge 0,61 g du produit de l'étape précédente, 0,15 ml de N-éthylpipérazine (d 0,899), 0,2 g de carbonate de potassium, 0,1 g de iodure de sodium dans 8 ml de DMF. On laisse réagir à 60°C pendant 4 heures puis on verse le mélange réactionnel dans l'eau et on extrait à l'acétate d'éthyle. La phase organique est séchée sur $Na_2SO_4$, filtrée et évaporée sous vide. On obtient 200 mg d'huile correspondante au produit du titre sous forme de base libre. On obtient la formation de l'oxalate.
On ajoute une solution d'acide oxalique en acétone dans une solution du produit en acétone. On filtre et on obtient 120 mg du produit du titre, sous forme de solide blanc.
P.f. = 192-196°C ; $[\alpha_D]$= +160°, c=0, 1166 wt% MeOH $^1$H NMR $\delta$
$^1$H NMR $\delta$ (ppm, dmso-d6): 1,16 (t,J= 7.1Hz, 3H), 2,67 - 2,86 (mb, 4H), 2, 87 - 3,14 (mb, 6H), 3,20-3,32 (m, 2H), 6,92 (s, 1 H), 7,01 (s, 1 H), 7,20 (s, 1 H), 7,25 (d, J=9.0Hz,1H), 7,53 (s, 1 H), 7,64 (d, J=9.0Hz,1H), 8,02 (sb, 1 H), 8,92 (s, 1 H), 10,07 (s, 1H).
LC/MS : $(M+H)^+$ = m/z 487 uma; rt = 4,7 minutes (méthodeA)

Méthode B :

1) Sous flux d'azote, on ajoute dans 40 ml de dichlorométhane anhydre refroidi dans un bain de glace, 1,23 g de $PCl_5$ puis on ajoute lentement 430 mg de l'acide de la Préparation 1. On laisse agir le mélange réactionnel à 0°C pendant 10 minutes puis à température ambiante pendant 3 heures.
2) D'autre part, on suspend sous flux d'azote 1 g du produit de la préparation 2, dans 40 ml de dichlorométhane puis on ajoute 1,3 ml de pyridine. On refroidit dans un bain de glace. On ajoute goutte à goutte la solution préparée en 1) et on agite à température ambiante pendant une heure.

On verse le mélange réactionnel dans l'eau et on extrait à l'acétate d'éthyle. La phase organique est lavée avec une solution saturée de $NaHCO_3$, séchée sur $Na_2SO_4$, filtrée et évaporée sous vide. On obtient 700 mg de solide orange, purifié sur colonne par flash chromatographie au moyen de l'éluant acétate d'éthyle 1 / méthanol 1 pour obtenir 440 mg de produit repris à l'isopropyléther de façon à obtenir 350 mg de produit du titre sous forme de base libre.
P.f. = 146-148°C; $[\alpha_D]$= +242°, c=0,1052. wt% in MeOH, ;

RMN: δ (ppm, dmso-d6): 0,98 (t, J= 7.2Hz, 3H), 2,29 (q, J= 7.2Hz, 2H), 2,37 (bm, 4H), 2,47 - 2,60 (m, **), 3,03*** (d, J= 15Hz, 1H),3,09*** (d, J= 15Hz, 1 H), 6,92 (d, J= 1.7Hz, 1 H), 7,01 (dd, J= 2.1 Hz and 0.7Hz, 1 H), 7,20 (d, J=1.7Hz, 1 H), 7,24 (dd, J= 8.8Hz and 2.0Hz, 1 H), 7,50 (d, J=2.0Hz, 1 H), 7,65 (d, J= 8.8Hz 1 H), 8,02 (d, J= 2.1 Hz, 1 H), 8,64 (s, 1 H), 10,71 (s, 1 H).

LC/MS : $(M+H)^+$ = m/z 487 uma; rt = 4,7 minutes (méthodeB)

Les composés selon l'invention ont fait l'objet d'études *in vivo.*

**Essai *in vivo***

Des rats mâles Crl CD BR (Charles River, Italie) pesant 150-175 g ont été logés dans une chambre à température (22±1°C), humidité (55 ± 10%) contrôlées et un cycle lumière-obscurité de 12 heures pendant au moins 7 jours avant leur utilisation. La nourriture et l'eau étaient disponibles *ad libitum.* L'alimentation a été retirée 18 heures avant le sacrifice des animaux. Les rats ont été sacrifiés par dislocation cervicale, l'estomac a été retiré chirurgicalement, ouvert le long de la courbure la plus faible, et placés dans une solution de Krebs (de composition (mM) : NaCl 118, 4; KCl 4,7 ; $CaCl_2$ 2,5 ; $NaH_2PO_4$ 3,7 ; $MgSO_4$ 1,2 ; $NaHCO_3$ 25 ; glucose 5,6). Le soin et le sacrifice des animaux ont été effectués selon le code éthique international de Sanofi-Aventis et les principes internationaux régissant le soin et le traitement des animaux de laboratoire (E.E.C. Directive 86/609, DJL358, 1, 12 décembre 1987). Des bandes d'environ 1 cm (5 mm de largeur) de fundus gastrique ont été coupées le long de l'axe longitudinal et suspendus dans 20 ml de bain rempli avec la solution de Krebs à 37°C et gazés avec un mélange gazeux de 95% $O_2$-5% $CO_2$. Les bandes ont été maintenues à une charge de repos de 1 g et après lavage de choline (précurseur de l'acétylcholine) 10 μM et 10 μM d'indométhacine (inhibiteur de prostaglandines synthétase) ont été ajoutés au milieu, pour réduire les contractions phasiques spontanées (Depoortere et al., Eur. J. Pharmacol. 515, 1-3, 160-168, 2003 ; Dass et al., Neurosciences 120, 443-453, 2003). Des contractions isotoniques ont été éveillées par stimulation par champ électrique. Deux électrodes de fil de platine ont été placées à la surface et au fond du bain d'organe et la stimulation par champ électrique a été réalisée par un stimulateur Power Lab (AD Instruments Pty Ltd. Castle Hill, Australie), couplé à un propulseur d'impulsion multiplex (Ugo Basile, Varese, Italie) (Fukuda et al. Scand. J. Gastroenterol. 12, 1209-1214, 2004). La stimulation supramaximale a été appliquée pour créer des contractions maximales (20 Hz, largeur de pulsation : 2 millisecondes ; 5 Volts ; trains de los toutes les 2 minutes, 150 mA). Ensuite, le courant a été réduit pour obtenir une stimulation submaximale (50% de réduction de la réponse contractile maximale). Les contractions ont été enregistrées par ordinateur avec un système d'enregistrement et d'analyse de données (Power Lab, Chart 5) relié à des transducteurs isotoniques (Ugo Basile, Varese, Italie) via des préamplificateurs (Octal Bridge Amp). Après stabilisation, des courbes cumulatives concentration-réponse de la ghréline (0,1 nM-1 μM) ont été tracées, avec et sans incubation (temps de contact : 30 mn) des molécules antagonistes. La stimulation par champ électrique supramaximale a été utilisée pour chaque bande comme référence (100 %) pour classer les réponses par substance test. La concentration agoniste produisant 50% d'effet maximal (EC50) a été calculée en utilisant un modèle logistique à quatre paramètres selon Ratkovsky et Reedy (Biometrics, 42, 575-582, 1986), avec ajustement par régression non linéaire en utilisant l'algorithme de Levenberg-Marquard dans le logiciel Everstat. Les valeurs pKB des antagonistes ont été calculées selon l'équation de Cheng-Prusoff (Kenakin et al. Competitive Antagonism, Pharmacologic Analysis of Drug-Receptor Interaction, 3e edition, 331-373, Philadelphie, New York ; Raven: Lippincott, 1997).

Les composés de formule (I) présentent une activité antagoniste du récepteur de la ghréline avec des $CI_{50}$ qui varient entre 5 $10^{-8}$ M et 1 $10^{-9}$M.

Par exemple, le composé de l'exemple 1 a montré une $CI_{50}$ de 2,2 $10^{-8}$M.

Il apparaît donc que les composés selon l'invention ont une activité antagoniste du récepteur de la ghréline.

Les composés de formule (I) ont démontré des propriétés pharmacologiques intéressantes telles la biodisponibilité, la toxicologie, de sélectivité, de métabolisme et pour le développement d'un médicament, en particulier de médicaments destinés à prévenir ou à traiter toute pathologie où le récepteur de la ghréline est impliqué.

Ainsi, selon un autre de ses aspects, l'invention a pour objet des médicaments qui comprennent un composé de formule (I) ou un sel d'addition de ce dernier à un acide pharmaceutiquement acceptable.

Ainsi, les composés selon l'invention peuvent être utilisés, chez l'homme ou chez l'animal, dans le traitement ou la prévention de différentes affections ghréline dépendantes. Ainsi, les composés selon l'invention peuvent être utilisés comme agents anorexiques, pour réguler l'appétit, la prise de repas et leur fréquence, ainsi que, à long terme, le poids, notamment la prise de poids faisant suite aux régimes diététiques ou thérapeutiques. Les composés selon l'invention sont donc particulièrement utiles pour la prévention ou le traitement de l'obésité, des troubles de l'appétit, du diabète, la surcharge pondérale et/ou de leurs effets.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, ainsi qu'au moins un excipient pharmaceutiquement acceptable.

Les dits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule ()I ci-dessus, ou son sel, peut être administré sous forme unitaire d'administration, en mélange avec des excipients pharmaceutiques classiques, aux animaux et aux êtres humains, pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Par voie orale, la dose de principe actif administrée par jour peut atteindre 0,1 à 100 mg/kg, en une ou plusieurs prises. Par voie parentérale, elle peut atteindre 0.01 à 10 mg/Kg/jour.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

Associations possibles

La présente invention concerne également les combinaisons d'un ou plusieurs composés(s) selon l'invention de formule générale (I) avec un ou plusieurs ingrédient(s) actif(s).

A titre d'ingrédient(s) actif(s) convenant auxdites combinaisons, on peut notamment citer les agents anti-obésité et antidiabétiques, ainsi que le rimonabant, la metformine ou les sulfonylurées.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables.

La présente invention, selon un autre de ses aspects, concerne également un composé de formule (I), ou un de ses sels pharmaceutiquement acceptables, pour le traitement des pathologies ci-dessus indiquées.

## Revendications

1. Composé répondant à la formule (I):

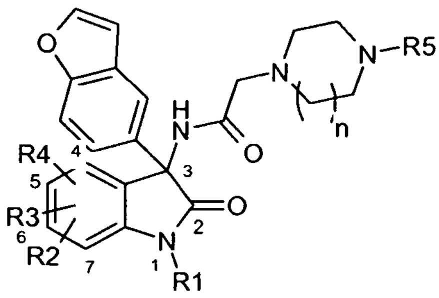

(I)

dans laquelle :

R1 représente un atome d'hydrogène ou un groupe (C1-6) alkyle, -C(=O)(C1-6) alkyle, -C(=O)aryle;
R2, R3, R4, identiques ou différents, situés sur l'une quelconque des positions disponibles du noyau phényle, représentent indépendamment un atome d'hydrogène, un atome d'halogène, CN , OH, un groupe (C1-6) alkyle éventuellement substitué par un atome d'halogène ou un OH ; perhalogéno(C1-3) alkyle, (C1-6) alcoxy, perhalogéno(C1-3) alcoxy , aminocarbonyle, (C1-6) alkylaminocarbonyle, di(C1-6) alkylaminocarbonyle, aryle, aryloxy , hétéroaryle ; le groupe aryle, aryloxy ou hétéroaryle pouvant éventuellement être substitué par un atome d'halogène, CN, OH ou un groupe (C1-6) alkyle , perhalogéno(C1-3) alkyle ou (C1-6) alcoxy ; étant entendu que au moins un des R2, R3, R4 est différent de H et que le groupe aryle, aryloxy ou hétéroaryle peut éventuellement être substitué par un atome d'halogène, CN, OH ou un groupe (C1-6) alkyle , perhalogéno (C1-3) alkyle, (C1-6) alcoxy;
R5 représente un groupe (C1-6) alkyle ou un (C2-6) alcényle;
n représente 1 ou 2 ;
sous forme de base ou de sel d'addition à un acide.

2. Composé selon la revendication 1 tel que dans la formule générale (I) :

R1 représente un atome d'hydrogène ou un groupe (C1-6) alkyle, -C(=O)(C1-6) alkyle, -C(=O)aryle;
R2, R3, R4, identiques ou différents, situés sur l'une quelconque des positions disponibles du noyau phényle, représentent indépendamment un atome d'hydrogène, un atome d'halogène, CN , OH, un groupe (C1-6) alkyle, perhalogéno(C1-3) alkyle, (C1-6) alcoxy, perhalogéno(C1-3) alcoxy , aminocarbonyle, (C1-6) alkylaminocarbonyle, di(C1-6) alkylaminocarbonyle, aryle, aryloxy ; hétéroaryle, étant entendu que au moins un des R2, R3, R4 est différent de H ;
R5 représente un groupe (C1-6) alkyle ;
n représente 1 ou 2 ;

sous forme de base ou de sel d'addition à un acide.

3. Composé selon la revendication 1 ou 2 tel que dans la formule générale (I) :

R1 représente un atome d'hydrogène ou un groupe -C(=O)(C1-6) alkyle, -C(=O)aryle, (C1-6) alkyle ;
R2, R3, R4, identiques ou différents, situés sur l'une quelconque des positions disponibles du noyau phényle, représentent indépendamment un atome d'hydrogène, un atome d'halogène, ou un groupe (C1-6) alkyle ou trifluorométhyle, étant entendu que au moins un des R2, R3, R4 est différent de H ;
R5 représente un groupe (C1-6) alkyle ;
n représente 1 ou 2 ;

sous forme de base ou de sel d'addition à un acide.

4. Composé selon l'une quelconque des revendications précédentes tel que dans la formule générale (I) :

R1 représente un atome d'hydrogène ou un groupe -C(=O)méthyle, -C(=O)phényle, méthyle ;
R2, R3, R4, identiques ou différents, situés sur l'une quelconque des positions disponibles du noyau phényle, représentent indépendamment un atome d'hydrogène, un atome d'halogène, ou un groupe méthyle ou trifluorométhyle, étant entendu que au moins un des R2, R3, R4 est différent de H ;
R5 représente un groupe méthyle, éthyle ou 2-propyle;
n représente 1 ou 2 ;

sous forme de base ou de sel d'addition à un acide.

**5.** Composé selon l'une quelconque des revendications précédentes choisi parmi le composé n°1 : (+) N-[4,6-Dichloro-3-(benzofuran-5-yl)-oxo-2,3-dihydro-1H-indol-3-yl]-2-(4-éthyl-pipérazin-1-yl)-acétamide ;
sous forme de base ou de sel d'addition à un acide.

**6.** Procédé de préparation d'un composé de formule (I) dans laquelle R1=H ou R1 représente un groupe alkyle selon l'une quelconque des revendications 1 à 5, **caractérisé en ce qu'**il comprend une étape consistant à faire réagir un composé de formule générale (V) :

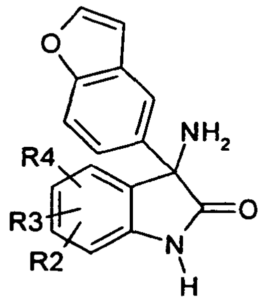

(V)

dans laquelle R2, R3 et R4 sont définis selon l'une quelconque des revendications 1 à 5.

**7.** Procédé selon la revendication 6 comprenant les étapes consistant à :

- faire réagir ledit composé de formule générale (V) avec un composé de formule générale (VI) :

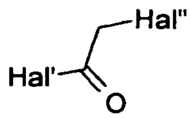

(VI)

dans laquelle Hal' et Hal", identiques ou différents représentent indépendamment un atome d'halogène ;
- puis faire réagir le composé de formule générale (III) obtenu

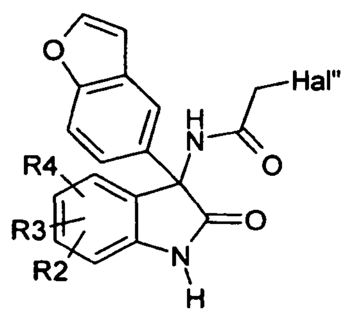

(III)

avec un composé de formule générale (IV) :

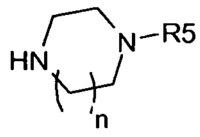

(IV)

dans lesquelles R2, R3, R4, R5 et n sont tels que définis en formule générale (I), et Hal" représente un atome d'halogène ;
- suivie éventuellement de l'étape consistant à faire réagir le produit de formule (I) obtenu dans laquelle R1 est égal à H, avec un composé de formule (II) :

R1-Hal (II)

dans laquelle R1, différent de H est défini comme en formule générale (I) et Hal représente un atome d'halogène.

8. Procédé selon la revendication 6 comprenant l'étape consistant à faire réagir ledit composé de formule générale (V) avec un composé de formule générale (VII) :

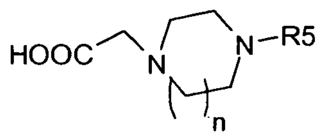

(VII)

dans laquelle R5 et n sont définis selon l'une quelconque des revendications 1 à 5, suivie éventuellement de l'étape consistant à faire réagir le produit de formule (I) obtenu dans laquelle R1 est égal à H, avec un composé de formule (II) :

R1-Hal (II)

dans laquelle R1, différent de H est défini comme en formule générale (I) et Hal représente un atome d'halogène.

9. Procédé de préparation d'un composé de formule (I) selon l'une quelconque des revendications 1 à 5, **caractérisé en ce qu'**il comprend une étape consistant à faire réagir un composé de formule générale (XVI) :

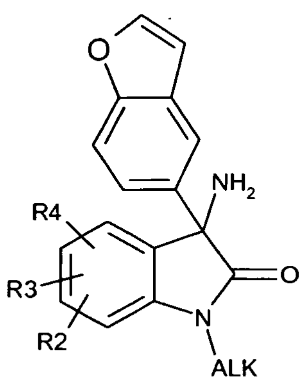

(XVI)

dans laquelle R2, R3 et R4 sont définis selon l'une quelconque des revendications 1 à 5 et ALK représente un groupe alkyle.

10. Procédé selon la revendication 9 comprenant l'étape consistant à faire réagir ledit composé de formule générale (XVI) avec un composé de formule générale (VII) :

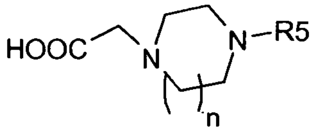

(VII)

dans laquelle R5 et n sont définis selon l'une quelconque des revendications 1 à 5.

**11.** Procédé selon l'une quelconque des revendications 6 à 10 comprenant l'étape ultérieure consistant à séparer le composé de formule générale (I) désiré.

**12.** Composé de formule (III) :

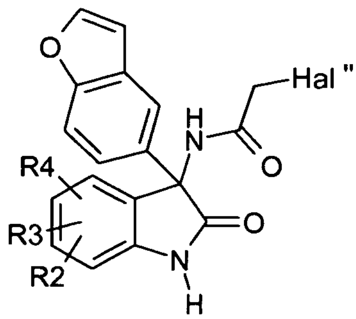

(III)

dans laquelle R2, R3 et R4 sont définis selon l'une quelconque des revendications 1 à 5 et Hal" représente un atome d'halogène.

**13.** Composé de formule générale (XVI) :

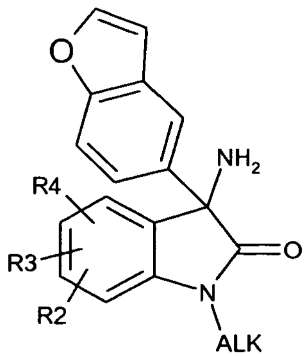

(XVI)

dans laquelle R2, R3 et R4 sont définis selon l'une quelconque des revendications 1 à 5 et ALK représente un groupe alkyle.

**14.** Médicament, **caractérisé en ce qu'**il comprend un composé de formule (I) selon l'une quelconque des revendications 1 à 5, ou un sel d'addition de ce composé à un acide pharmaceutiquement acceptable.

**15.** Composition pharmaceutique, **caractérisée en ce qu'**elle comprend un composé de formule (I) selon l'une quelconque des revendications 1 à 5, ou un sel pharmaceutiquement acceptable.

**16.** Utilisation d'un composé selon l'une quelconque des revendications 1 à 5 pour la préparation d'un médicament destiné à la prévention ou au traitement de l'obésité, du diabète, des troubles de l'appétit et de la surcharge pondérale.

17. Composé selon l'une quelconque des revendications 1 à 5 pour la prévention ou le traitement de l'obésité, du diabète, des troubles de l'appétit et de la surcharge pondérale.

18. Combinaison comprenant un ou plusieurs composés(s) selon l'une quelconque des revendications 1 à 5 avec un ou plusieurs ingrédient(s) actif(s).

**Patentansprüche**

1. Verbindung der Formel (I):

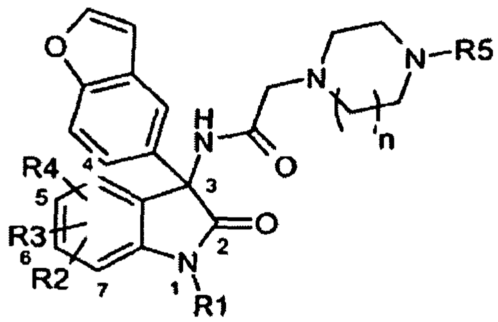

(I)

mit den folgenden Bedeutungen:

R1 bedeutet ein Wasserstoffatom oder eine (C1-6)-Alkylgruppe, -C(=O)(C1-6)-Alkylgruppe oder -C(=O)-Arylgruppe;
R2, R3, R4, die gleich oder verschieden sind und sich an einer beliebigen verfügbaren Position des Phenylrings befinden, bedeuten unabhängig ein Wasserstoffatom, ein Halogenatom, CN, OH, eine gegebenenfalls durch ein Halogenatom oder OH substituierte (C1-6)-Alkylgruppe; Perhalogen-(C1-3)-alkyl, (C1-6)-Alkoxy, Perhalogen-(C1-3)-alkoxy, Aminocarbonyl, (C1-6)-Alkylaminocarbonyl, Di-(C1-6)-Alkylaminocarbonyl, Aryl, Aryloxy, Heteroaryl; wobei die Aryl-, Aryloxy- oder Heteroarylgruppe gegebenenfalls durch ein Halogenatom, CN, OH oder eine (C1-6)-Alkylgruppe, Perhalogen(C1-3)-alkylgruppe oder (C1-6)-Alkoxygruppe substituiert sein kann; mit der Maßgabe, dass mindestens einer der Reste R2, R3, R4 nicht H bedeutet und dass die Aryl-, Aryloxy- oder Heteroarylgruppe gegebenenfalls durch ein Halogenatom, CN, OH oder eine (C1-6)-Alkylgruppe, Perhalogen(C1-3)-alkylgruppe, (C1-6)-Alkoxygruppe substituiert sein kann;
R5 bedeutet eine (C1-6)-Alkylgruppe oder eine (C2-6)-Alkenylgruppe;
n bedeutet 1 oder 2;

in Basenform oder in Form eines Säureadditionssalzes.

2. Verbindung nach Anspruch 1, wobei die Symbole in der allgemeinen Formel (I) die folgenden Bedeutungen aufweisen:

R1 bedeutet ein Wasserstoffatom oder eine (C1-6)-Alkylgruppe, -C(=O)(C1-6)-Alkylgruppe, -C(=O)-Arylgruppe;
R2, R3, R4, die gleich oder verschieden sind und sich an einer beliebigen verfügbaren Position des Phenylrings befinden, bedeuten unabhängig ein Wasserstoffatom, ein Halogenatom, CN, OH, eine (C1-6)-Alkylgruppe; Perhalogen(C1-3)-alkylgruppe, (C1-6)-Alkoxygruppe, Perhalogen(C1-3)-alkoxygruppe, Aminocarbonylgruppe, (C1-6)-Alkylamino-carbonylgruppe, Di(C1-6)-Alkylaminocarbonylgruppe, Arylgruppe, Aryloxygruppe; Heteroaryl, mit der Maßgabe, dass mindestens einer der Reste R2, R3, R4 nicht H bedeutet;
R5 bedeutet eine (C1-6)-Alkylgruppe;
n bedeutet 1 oder 2;

in Basenform oder in Form eines Säureadditionssalzes.

3. Verbindung nach Anspruch 1 oder 2, wobei die Symbole in der allgemeinen Formel (I) die folgenden Bedeutungen

aufweisen:

R1 bedeutet ein Wasserstoffatom oder eine -C(=O)(C1-6)-Alkylgruppe, -C(=O)-Arylgruppe, (C1-6)-Alkylgruppe;
R2, R3, R4, die gleich oder verschieden sind und sich an einer beliebigen verfügbaren Position des Phenylrings befinden, bedeuten unabhängig ein Wasserstoffatom, ein Halogenatom, oder eine ($C_1$-6)-Alkylgruppe oder Trifluormethylgruppe, mit der Maßgabe, dass mindestens einer der Reste R2, R3, R4 nicht H bedeutet;
R5 bedeutet eine (C1-6)-Alkylgruppe;
n bedeutet 1 oder 2;

in Basenform oder in Form eines Säureadditionssalzes.

**4.** Verbindung nach einem der vorhergehenden Ansprüche, wobei die Symbole in der allgemeinen Formel (I) die folgenden Bedeutungen aufweisen:

R1 bedeutet ein Wasserstoffatom oder eine -C(=O)-Methylgruppe, -C(=O)-Phenylgruppe, Methylgruppe;
R2, R3, R4, die gleich oder verschieden sind und sich an einer beliebigen verfügbaren Position des Phenylrings befinden, bedeuten unabhängig ein Wasserstoffatom, ein Halogenatom oder eine Methyl-oder Trifluormethylgruppe, mit der Maßgabe, dass mindestens einer der Reste R2, R3, R4 nicht H bedeutet;
R5 bedeutet eine Methylgruppe, Ethylgruppe oder 2-Propylgruppe;
n bedeutet 1 oder 2;

in Basenform oder in Form eines Säureadditionssalzes.

**5.** Verbindung nach einem der vorhergehenden Ansprüche, ausgewählt aus der Verbindung Nr. 1: (+) N-[4,6-Dichlor-3-(benzofuran-5-yl)-2-oxo-2,3-dihydro-1H-indol-3-yl]-2-(4-ethylpiperazin-1-yl)acetamid;
in Basenform oder in Form eines Säureadditionssalzes.

**6.** Verfahren zur Herstellung einer Verbindung der Formel (I), in der R1 = H oder R1 eine Alkylgruppe bedeutet, nach einem der Ansprüche 1 bis 5, **dadurch gekennzeichnet, dass** es den Schritt umfasst, der darin besteht, dass man eine Verbindung der allgemeinen Formel (V),

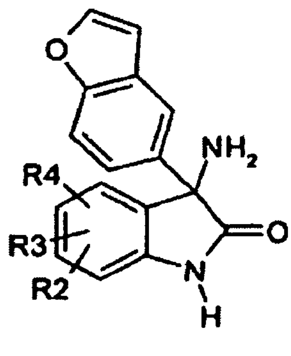

(V)

in der R2, R3 und R4 wie in einem der Ansprüche 1 bis 5 definiert sind,
umsetzt.

**7.** Verfahren nach Anspruch 6, umfassend die Schritte, die darin bestehen, dass man

- die Verbindung der allgemeinen Formel (V) mit einer Verbindung der allgemeinen Formel (VI),

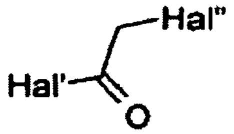

(VI)

in der Hal’ und Hal", die gleich oder verschieden sind, unabhängig ein Halogenatom bedeuten, umsetzt;
- dann die erhaltene Verbindung der allgemeinen Formel (III)

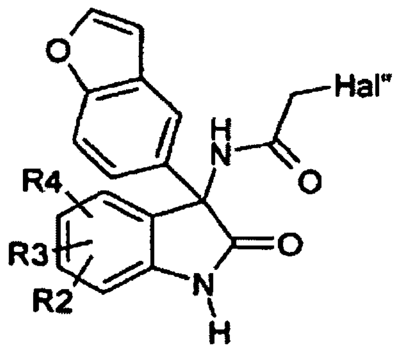

(III)

mit einer Verbindung der allgemeinen Formel (IV),

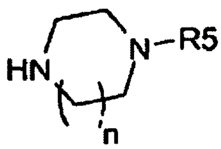

(IV)

in der R2, R3, R4, R5 und n wie in der allgemeinen Formel (I) definiert sind und Hal" ein Halogenatom bedeutet, umsetzt;
- gegebenenfalls gefolgt von dem Schritt, der darin besteht, dass man das erhaltene Produkt der Formel (I), in der R1 gleich H ist, mit einer Verbindung der Formel (II),

R1-Hal (II)

in der R1, das nicht H bedeutet, wie in der allgemeinen Formel (I) definiert ist und Hal ein Halogenatom bedeutet,

umsetzt.

8. Verfahren nach Anspruch 6, umfassend den Schritt, der darin besteht, dass man die Verbindung der allgemeinen Formel (V) mit einer Verbindung der allgemeinen Formel (VII),

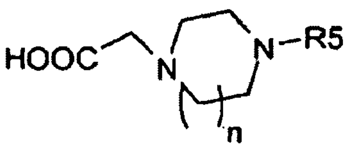

(VII)

in der R5 und n wie in einem der Ansprüche 1 bis 5 definiert sind, umsetzt,

- gegebenenfalls gefolgt von dem Schritt, der darin besteht, dass man das erhaltene Produkt der Formel (I), in der R1 gleich H ist, mit einer Verbindung der Formel (II),

R1-Hal (II)

in der R1, das nicht H bedeutet, wie in der allgemeinen Formel (I) definiert ist und Hal ein Halogenatom bedeutet,

umsetzt.

**9.** Verfahren zur Herstellung einer Verbindung der Formel (I) nach einem der Ansprüche 1 bis 5, **dadurch gekennzeichnet, dass** es einen Schritt umfasst, der darin besteht, dass man eine Verbindung der allgemeinen Formel (XVI),

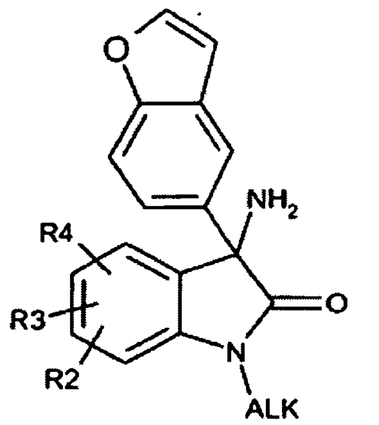

(XVI)

in der R2, R3 und R4 wie in einem der Ansprüche 1 bis 5 definiert sind und ALK eine Alkylgruppe bedeutet, umsetzt.

**10.** Verfahren nach Anspruch 9, das den Schritt umfasst, der darin besteht, dass man die Verbindung der allgemeinen Formel (XVI) mit einer Verbindung der allgemeinen Formel (VII),

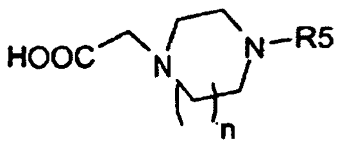

(VII)

in der R5 und n wie in einem der Ansprüche 1 bis 5 definiert sind,
umsetzt.

**11.** Verfahren nach einem der Ansprüche 6 bis 10, das den weiteren Schritt umfasst, der darin besteht, dass man die gewünschte Verbindung der allgemeinen Formel (I) abtrennt.

**12.** Verbindung der Formel (III),

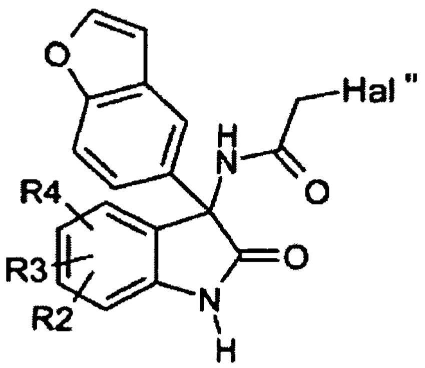

(III)

in der R2, R3 und R4 wie in einem der Ansprüche 1 bis 5 definiert sind und Hal" ein Halogenatom bedeutet.

**13.** Verbindung der allgemeinen Formel (XVI),

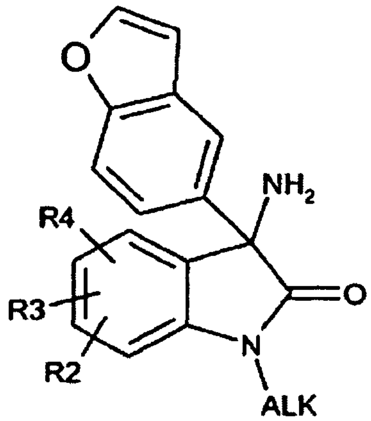

(XVI)

in der R2, R3 und R4 wie in einem der Ansprüche 1 bis 5 definiert sind und ALK eine Alkylgruppe bedeutet.

**14.** Arzneimittel, **dadurch gekennzeichnet, dass** es eine Verbindung der Formel (I) nach einem der Ansprüche 1 bis 5 oder ein Additionssalz dieser Verbindung mit einer pharmazeutisch unbedenklichen Säure umfasst.

**15.** Pharmazeutische Zusammensetzung, **dadurch gekennzeichnet, dass** sie eine Verbindung der Formel (I) nach einem der Ansprüche 1 bis 5 oder ein pharmazeutisch unbedenkliches Salz umfasst.

**16.** Verwendung einer Verbindung nach einem der Ansprüche 1 bis 5 für die Herstellung eines Arzneimittels für die Vorbeugung oder Behandlung von Adipositas, Diabetes, Appetitstörungen und Übergewicht.

**17.** Verbindung nach einem der Ansprüche 1 bis 5 zur Vorbeugung oder Behandlung von Adipositas, Diabetes, Appetitstörungen und Übergewicht.

**18.** Kombination, die eine Verbindung oder mehrere Verbindungen nach einem der Ansprüche 1 bis 5 zusammen mit einem weiteren Wirkstoff bzw. weiteren Wirkstoffen umfasst.

## Claims

**1.** Compound corresponding to formula (I):

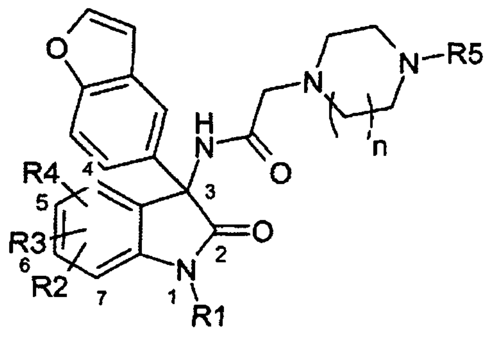

(I)

in which:

R1 represents a hydrogen atom or a (C1-6)alkyl, - C (=O) (C1-6) alkyl or -C (=O) aryl group;
R2, R3 and R4, which may be identical or different, located on any of the available positions of the phenyl nucleus, independently represent a hydrogen atom, a halogen atom, CN, OH, a (C1-6)alkyl group optionally substituted with a halogen atom or an OH; perhalo(C1-3)alkyl, (C1-6) alkoxy, perhalo (C1-3) alkoxy, aminocarbonyl, (C1-6)alkylaminocarbonyl, di(C1-6)alkylaminocarbonyl, aryl, aryloxy; heteroaryl; the aryl, aryloxy or heteroaryl group possibly being optionally substituted with a halogen atom, CN, OH or a (C1-6)alkyl, perhalo (C1-3)alkyl or (C1-6)alkoxy group; it being understood that at least one from among R2, R3 and R4 is other than H and that the aryl, aryloxy or heteroaryl group may be optionally substituted with a halogen atom, CN, OH or a (C1-6)alkyl, perhalo(C1-3)alkyl or (C1-6)alkoxy group;
R5 represents a (C1-6)alkyl or (C2-6)alkenyl group;
n represents 1 or 2;

in the form of the base or of an acid-addition salt.

**2.** Compound according to Claim 1, such that, in the general formula (I):

R1 represents a hydrogen atom or a (C1-6)alkyl, - C (=O) (C1-6) alkyl or -C(=O)aryl group;
R2, R3 and R4, which may be identical or different, located on any of the available positions of the phenyl nucleus, independently represent a hydrogen atom, a halogen atom, CN, OH or a (C1-6)alkyl, perhalo(C1-3) alkyl, (C1-6)alkoxy, perhalo(C1-3)alkoxy, aminocarbonyl, (C1-6)alkylaminocarbonyl, di(C1-6)alkylaminocarbonyl, aryl, aryloxy or heteroaryl group, it being understood that at least one from among R2, R3 and R4 is other than H;
R5 represents a (C1-6)alkyl group;
n represents 1 or 2;

in the form of the base or of an acid-addition salt.

**3.** Compound according to Claim 1 or 2, such that, in the general formula (I):

R1 represents a hydrogen atom or a -C(=O)(C1-6)alkyl, -C (=O) aryl or (C1-6) alkyl group;
R2, R3 and R4, which may be identical or different, located on any of the available positions of the phenyl nucleus, independently represent a hydrogen atom, a halogen atom, or a (C1-6)alkyl or trifluoromethyl group, it being understood that at least one from among R2, R3 and R4 is other than H;
R5 represents a (C1-6)alkyl group;
n represents 1 or 2;

in the form of the base or of an acid-addition salt.

**4.** Compound according to any one of the preceding claims, such that, in the general formula (I):

R1 represents a hydrogen atom or a -C(=O)methyl, -C(=O)phenyl or methyl group;
R2, R3 and R4, which may be identical or different, located on any of the available positions of the phenyl

nucleus, independently represent a hydrogen atom, a halogen atom, or a methyl or trifluoromethyl group, it being understood that at least one from among R2, R3 and R4 is other than H;
R5 represents a methyl, ethyl or 2-propyl group;
n represents 1 or 2;

in the form of the base or of an acid-addition salt.

**5.** Compound according to any one of the preceding claims, chosen from compound No. 1: (+)-N-[4,6-dichloro-3-(benzofuran-5-yl)-2-oxo-2,3-dihydro-1H-indol-3-yl]-2-(4-ethylpiperazin-1-yl)acetamide;
in the form of the base or of an acid-addition salt.

**6.** Process for preparing a compound of formula (I) in which R1 = H or R1 represents an alkyl group according to any one of Claims 1 to 5, **characterized in that** it comprises a step that consists in reacting a compound of general formula (V):

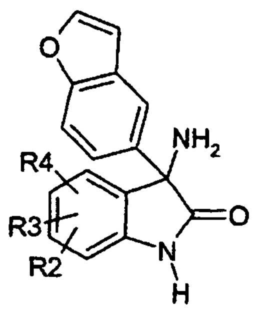

(V)

in which R2, R3 and R4 are as defined according to any one of Claims 1 to 5.

**7.** Process according to Claim 6, comprising the steps consisting in:

- reacting the said compound of general formula (V) with a compound of general formula (VI):

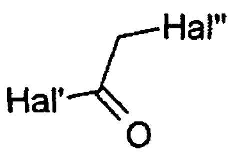

(VI)

in which Hal' and Hal", which may be identical or different, independently represent a halogen atom;
- and then in reacting the compound of general formula (III) obtained

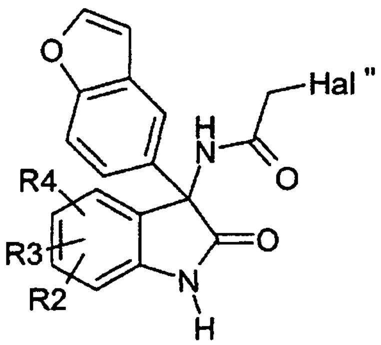

(III)

with a compound of general formula (IV):

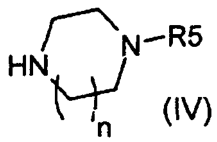

in which R2, R3, R4, R5 and n are defined as in the general formula (I) and Hal" represents a halogen atom;
- optionally followed by the step that consists in reacting the product of formula (I) obtained, in which R1 is equal to H, with a compound of formula (II):

R1-Hal (II)

in which R1, which is other than H, is defined as in the general formula (I) and Hal represents a halogen atom.

**8.** Process according to Claim 6, comprising the step that consists in reacting the said compound of general formula (V) with a compound of general formula (VII):

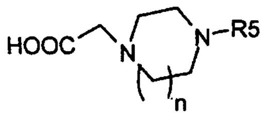

(VII)

in which R5 and n are as defined according to any one of Claims 1 to 5;
optionally followed by the step that consists in reacting the product of formula (I) obtained, in which R1 is equal to H, with a compound of formula (II):

R1-Hal (II)

in which R1, which is other than H, is defined as in the general formula (I) and Hal represents a halogen atom.

**9.** Process for preparing a compound of formula (I) according to any one of Claims 1 to 5, **characterized in that** it comprises a step that consists in reacting a compound of general formula (XVI):

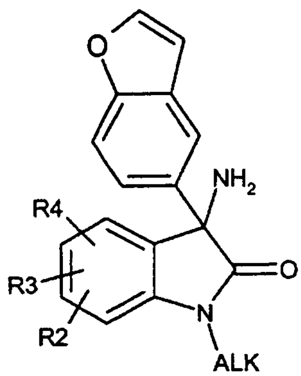

(XVI)

in which R2, R3 and R4 are defined according to any one of Claims 1 to 5 and ALK represents an alkyl group.

**10.** Process according to Claim 9, comprising the step that consists in reacting the said compound of general formula (XVI) with a compound of general formula (VII):

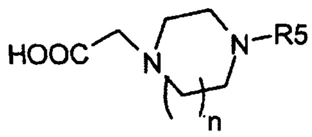

(VII)

in which R5 and n are defined according to any one of Claims 1 to 5.

**11.** Process according to any one of Claims 6 to 10, comprising the subsequent step that consists in separating out the desired compound of general formula (I) .

**12.** Compound of formula (III):

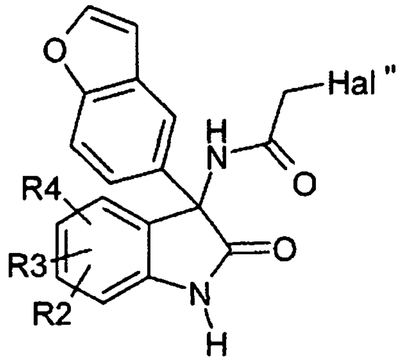

(III)

in which R2, R3 and R4 are defined according to any one of Claims 1 to 5 and Hal" represents a halogen atom.

**13.** Compound of general formula (XVI):

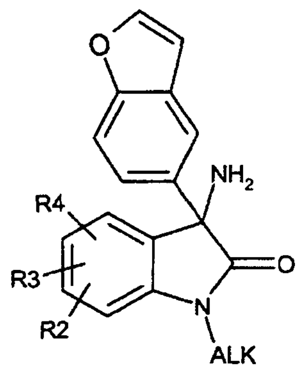

(XVI)

in which R2, R3 and R4 are defined according to any one of Claims 1 to 5 and ALK represents an alkyl group.

**14.** Medicament, **characterized in that** it comprises a compound of formula (I) according to any one of Claims 1 to 5, or an addition salt of this compound with a pharmaceutically acceptable acid.

**15.** Pharmaceutical composition, **characterized in that** it comprises a compound of formula (I) according to any one of Claims 1 to 5, or a pharmaceutically acceptable salt.

**16.** Use of a compound according to any one of Claims 1 to 5 for the preparation of a medicament for preventing or

treating obesity, diabetes, appetite disorders and excess weight.

17. Compound according to any one of Claims 1 to 5, for preventing or treating obesity, diabetes, appetite disorders and excess weight.

18. Combination comprising one or more compounds according to any one of Claims 1 to 5 with one or more active ingredient(s).

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2714378
- WO 03008407 A


### Littérature non-brevet citée dans la description


- **KOJIMA M et al.** *Nature,* 1999, vol. 402, 656-60
- **SMITH R.G. et al.** *Trends in Endocrinology and Metabolism,* 2005, vol. 16 (9
- **UENO, N. et al.** *Endocrinology,* 2004, vol. 145, 4176-4184
- **KIM, M.S. et al.** *Int. J. Obes. Relat. Metab. Disord.,* 2004, vol. 28, 1264-1271
- **BARAZZONI, R. et al.** *Am. J. Physiol. Endocrinol. Metab.,* 2004, vol. 288, E228-E235
- *Horm. Res.,* 2003, vol. 59 (3), 109-17
- **WREN A.M. et al.** *J. Clin. Endocrinol. Metab.,* 2001, vol. 86, 5992-5
- **DRUCE M.R. et al.** *Int. J. Obes.,* 2005, vol. 29, 1130-6
- **WYNNE K. et al.** *J. Am. Soc. Nephrol.,* 2005, vol. 16, 2111-8
- **PRADO, C.L. et al.** *Proc. Natl. Acad. Sci. USA,* 2004, vol. 101, 2924-2929
- **DEZAKI, K. et al.** *Diabètes,* 2004, vol. 53, 3142-3151
- **SUN Y. et al.** *Cell Metabolism,* 2006, vol. 3, 379-386
- **GREEN et al.** Protective Groups in Organic Synthesis. John Wiley & Sons, Inc,
- **J. MARCH.** Advances in Organic Chemistry. Wiley Interscience, 310-316
- E.E.C. Directive 86/609. *DJL358,* 12 Décembre 1987, vol. 1
- **DEPOORTERE et al.** *Eur. J. Pharmacol.,* 2003, vol. 515 (1-3), 160-168
- **DASS et al.** *Neurosciences,* 2003, vol. 120, 443-453
- **FUKUDA et al.** *Scand. J. Gastroenterol.,* 2004, vol. 12, 1209-1214
- **RATKOVSKY ; REEDY.** *Biometrics,* 1986, vol. 42, 575-582
- **KENAKIN et al.** Competitive Antagonism, Pharmacologic Analysis of Drug-Receptor Interaction. Raven: Lippincott, 1997, 331-373